# EUROPEAN PATENT SPECIFICATION

(11) **EP 0 064 322 B1**
(45) Date of publication and mention of the grant of the patent: **22.05.1985**
(21) Application number: 82200654.0
(22) Date of filing: 24.07.1980
(51) Int. Cl.: C07C 143/828, C07C 143/78, C07C 143/70, C07C 161/04, C07C 149/44, C07C 147/06, C07C 147/14, C07C 93/14

(54) **Benzenesulfonyl isocyanates, isothiocyanates, chlorides, and sulfonamides having a fluorinated substituent**
Benzolsulfonylisocyanate, Benzolsulfonylisothiocyanate, Benzolsulfonylchloride und Benzolsulfonamide, die fluorierte Substituenten enthalten
Isocyanates et isothiocyanates de benzènesulphonyles, benzène-sulfochlorures et benzènesulfonamides contenant des substituants fluorés

(30) Priority: 26.07.1979 US 60869; 30.05.1980 US 152021
(43) Date of publication of application: 10.11.1982
(62) Divisional of application: 80302536.0
(73) Proprietor: E.I. DU PONT DE NEMOURS AND COMPANY, Wilmington Delaware 19898 (US)
(72) Inventor: Adams, John Benjamin, Jr., Auburn Hockessin Delaware 19707 (US)
(74) Representative: Hildyard, Edward Martin

## Description

This invention relates to benzenesulfonyl isocyanates and isothiocyanates having a fluorinated substituent in the aromatic ring, and to the corresponding sulfonamides and sulfonyl chlorides which serve as precursors.

The novel compounds of this invention have the general formula 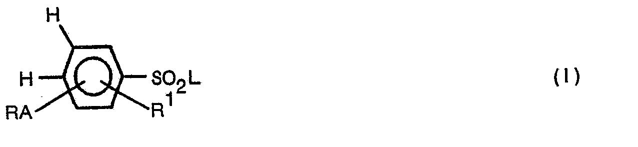
wherein R is CF₃, CHF₂, CH₂CF₃ or CF₂CHFG, where G is F, Cl, Br or CF3;
A is 0 or S(O)ₙ, where n is 0, 1 or 2;
R¹ is H, F, Cl, Br or CH₃; and
L is Cl, NH₂, NCO or NCS.

In our preferred compounds R¹ is H or CI and A is 0 or S. Compounds of particular interest are the following:
5-chloro-2-trifluoromethoxybenzenesulfonyl isocyanate;
2-chloro-5-trifluoromethoxybenzenesulfonyt isocyanate;
2-(1,1,2;2-tetrafluoroethoxy)benzenesulfonyl isocyanate;

2-difluoromethoxybenzenesulfonyl isocyanate; as well as the corresponding sulfonamides and sulfonyl halides having the same substitution pattern in the aromatic ring.

The isocyanates and isothiocyanates of general formula (I) are valuable intermediates for the preparation of the sulfonylurea derivatives disclosed in or copending EP-A-0,023,422, to which the reader is referred. The latter compounds have potent herbicidal and growth regulant effects. The particular nature and orientation of the substituents RA and R¹ in the compounds of the present invention result in sulfonylurea final products having unexpectedly valuable properties. The preferred isocyanates listed above can give rise to sulfonylureas having particularly outstanding herbicidal properties.

### Synthesis

Since not all of the compounds included within Formula (I) can be made by the same synthesis scheme, Formula (I) is divided into compound groups II and III, synthesis for each of which is discussed separately; compound groups II and III are:
(Haloalkoxy)benzenesulfonyl derivatives 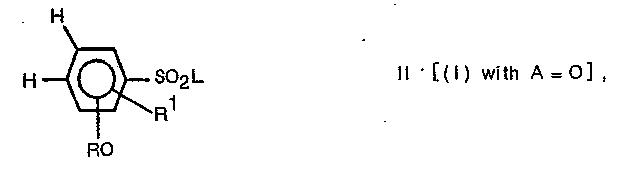 and
(Haloalkylthio, haloalkylsulfinyl, and haloalkylsulfonyl)benzenesulfonyl derivatives 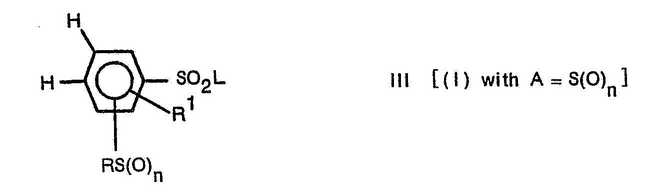 (Haloalkoxy)benzenesulfonyl derivatives 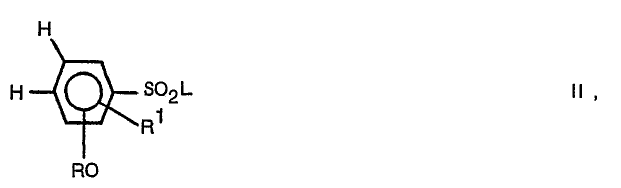 wherein the substituents are defined as for Formula (I).
(Haloalkoxy)benzene derivatives (1) as started materials for preparation of II can be made by methods well known in the art.

(Trifluoromethoxy)benzene derivatives can be made by the method of Sheppard [J. Org. Chem. 29, 1 (1964)], e.g.:

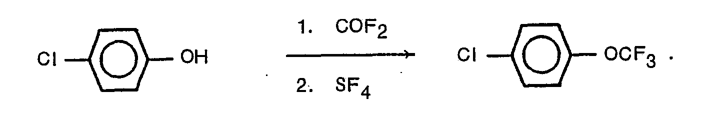

(Tetrahaloethoxy)benzene derivatives can be made by the method of England et al. [J. Am. Chem. Soc. 82, 5116 (1960)], which also applies to the hexafluoropropoxy compounds, e.g.

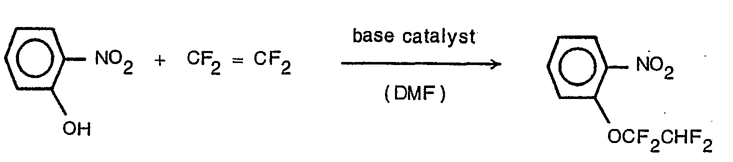

The latter compound can be purchased from Fairfield Chemical Co., Blythewood, S.C.

(Trifluoroethoxy)benzene derivatives can be made by reaction of trifluoroethanol with an activated aromatic halide, e.g.:

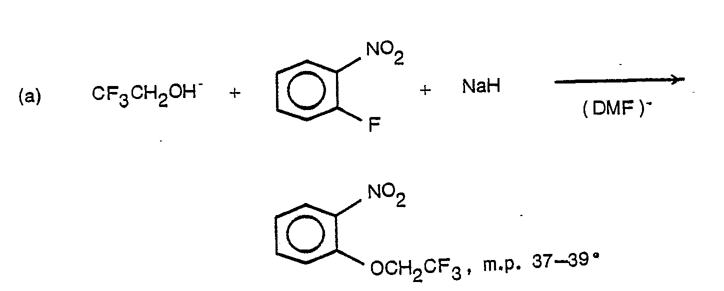

In reaction (a) sodium hydride and trifluoroethanol are mixed in an aprotic solvent, such as dimethylformamide (DMF), dioxane or tetrahydrofuran (THF), with 2-(fluoro or chloro)-1-nitrobenzene. The reaction proceeds to completion at ambient temperature. Heat may be applied (e.g., with a steam bath) if desired to speed the reaction to completion. The product is isolated by diluting the reaction mixture with water, extracting with an organic water-immiscible solvent and evaporation of the solvent. This reaction is similar to that described in Japanese Patent 5 2057-320.

(Difluoromethoxy)benzene derivatives can be made by the method of Yagupol'skii et al. [Chem. Abstr. 70, 96318d (1969)] e.g.:

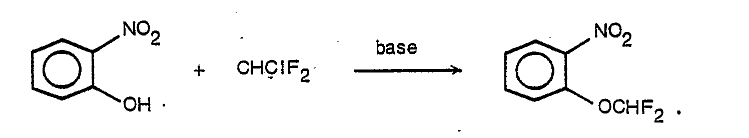

Preparation of compound II is shown schematically, first for the case wherein R is CF₃ or CF₃CH₂ and A is 0 or S:

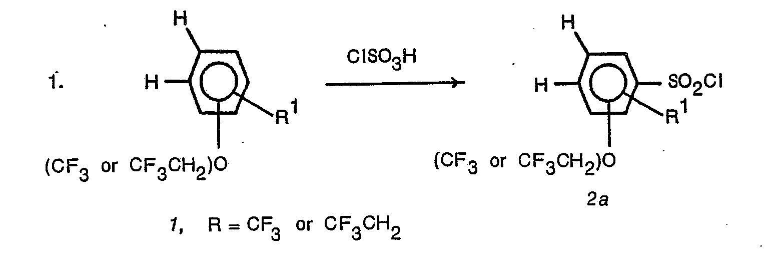

Chlorosulfonation of aromatic substrates is well known (e.g., L. F. Fieser and M. Fieser, "Advanced Organic Chemistry", 696―698, Reinhold, New York, 1961). The chlorosulfonation can be accomplished by addition of the trifluoromethoxy compound to the chlorosulfonic acid or vice versa, optionally in the presence of a cosolvent, such as an alkane or chlorinated alkane (e.g., hexane, 1-chlorobutane, methylene chloride, etc.). The reaction temperature is not critical, with a range of about -5° to 50° operable and ambient temperature (e.g. 20 to 30°) preferred, for convenience. At ambient temperature some hydrolysis of the trifluoromethoxy group occurs. At lower temperatures, chlorosulfonation occurs more slowly with less of the hydrolysis, while at higher temperatures, chlorosulfonation occurs more rapidly with more accompanying hydrolysis. Reaction time at ambient temperature is about 1 to 24 hours, depending on the exact substrate being chlorosulfonated, with an overnight period (about 16 hours) satisfactory. Chlorosulfonation of the tetrahaloethoxy, hexafluoropropoxy and difluoromethoxy compounds is more difficult to control without hydrolysis of the haloalkoxy group than is chlorosulfonation of the trifluoromethoxy or trifluoroethoxy compounds.

The aromatic sulfonyl chloride is conveniently isolated from the reaction mixture by pouring the mixture into ice water, followed by extraction with a water-immiscible organic solvent in which the aromatic sulfonyl chloride is soluble. Such solvents include 1-chlorobutane, methylene chloride, 1,2-dichloroethane, ethyl acetate, toluene and diethyl ether. The solution of the sulfonyl chloride can be dried and evaporated to provide the sulfonyl chloride, which can be further purified by distillation, preferably in vacuum to suppress any thermally dependent decomposition. Alternatively, the solution of the sulfonyl chloride can be used directly in reaction with ammonia in the next step, preparation of the sulfonamide.

Chlorosulfonation of 1 can produce isomeric mixtures, e.g.:

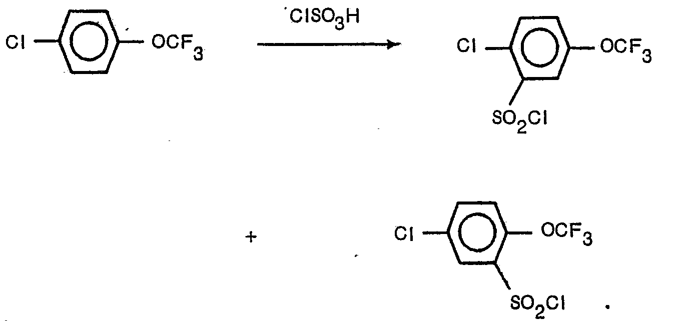

Such isomeric mixtures can be separated by conventional routes (e.g., fractional distillation, chromatography) or used without separation. In the latter case, isomeric mixtures of sulfonamides, sulfonyl isocyanates and thiocyanates, sulfonylureas and sulfonylthioureas are formed in the subsequent reactions. Similarly, the isomeric mixtures of intermediates formed further in the synthesis sequence can be separated or used as isomeric mixtures; isomeric mixtures of product sulfonyl (ureas and thioureas) can be used as herbicides or separated and used as individual compounds.

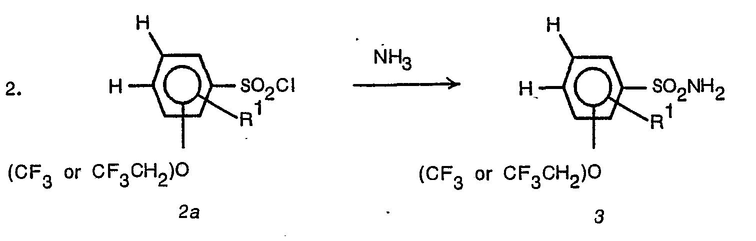

Conversion of sulfonyl chlorides to sulfonamides is well known (e.g., L. Fieser and M. Fieser, op. cit., 699). It is convenient to dissolve the sulfonyl chloride 2a in an inert solvent, e.g. toluene, ethyl acetate, tetrahydrofuran, etc., and sparge in gaseous ammonia until the formation of ammonium chloride (insoluble in the solvent) ceases. Temperature is not critical and can range from about -20° to the boiling point of the solvent. For convenience, ambient temperatures are preferred.

The product can be isolated from the reaction mixture by evaporation and treatment of the residue with water to remove ammonium chloride. If the product precipitates during reaction it can be removed by filtration of the reaction mixture and washing with water. If the product remains in solution in the reaction mixture and the solvent is water-immiscible, the mixture can be washed with water and the product obtained by evaporation of the solvent. If the product remains in solution in the reaction mixture and the solvent is water-miscible, the product can be precipitated by addition of water, then recovered by filtration.

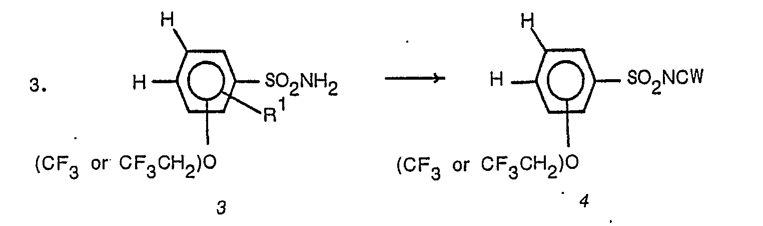

The sulfonamide 3 is converted to the sulfonyl isocyanate or sulfonyl isothiocyanate 4 (W = 0 or S).

Sulfonyl isocyanates can be made by the method of Ulrich et al. [J. Org. Chem. 34, 3200 (1969)]:

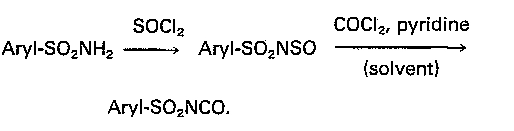

The sulfonamide is boiled under reflux with an excess of thionyl chloride, which functions as a reactant and solvent. The reaction is continued until the sulfonamide protons are undetectable in the proton resonance spectrum. An overnight reaction period (about 16 hours) is generally sufficient. The thionyl chloride is evaporated and the residue dissolved in an inert solvent, such as toluene, benzene, xylene, etc., treated with a catalytic amount of a suitable base, e.g. pyridine, then with at least one equivalent of phosgene. The mixture is heated to about 60-140°C, with 80-100^{*} preferred. Conversion to the isocyanate is substantially complete within about to 3 hours. The mixture containing the sulfonyl isocyanate can be used directly for the next reaction step (formation of sulfonylurea) or the sulfonyl isocyanate can be isolated in purified form by filtration and evaporation of the filtrate, optionally followed by vacuum distillation.

Sulfonyl isocyanates can also be made by mixing the sulfonamide, an alkyl isocyanate (e.g. butyl isocyanate) and a catalytic amount of 1,4-diaza[2.2.2]-bicyclooctane (DABCO), in xylene or other inert solvent of sufficiently high boiling point (e.g. >135°), heating to about 135°, and adding phosgene until an excess is present (indicated by a drop in boiling point). The mixture is further heated and excess phosgene driven off. After the mixture is cooled and filtered from insoluble material, the solvent, alkyl isocyanate and excess phosgene are evaporated, leaving the crude sulfonyl isocyanate, optionally .purified further by vacuum distillation.

Sulfonyl isothiocyanates can be made by the method of Hartke [Chem. Abstr. 64, 15783e (1966)]:

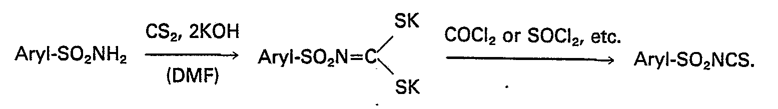

The sulfonamide in dimethylformamide (DMF) is treated with an equivalent of carbon disulfide and 2 equivalents of powdered potassium hydroxide at about 35°. The mixture is stirred (about 1-8 hours) until solution is substantially complete, then diluted with an aprotic solvent, such as ethyl acetate, to precipitate the intermediate dipotassium salt shown. The latter is separated by filtration from the reaction mixture, suspended in an inert solvent, such as toluene, and treated with 2 moles of phosgene (or thionyl chloride) at about 0°. The mixture is allowed to warm to ambient temperature, filtered, and the sulfonyl isothiocyanate used in solution for the next reaction step (formation of sulfonylthiourea) or isolated by evaporation of solvent. The sulfonyl isothiocyanates may dimerize or trimerize in some cases, but the dimers and trimers still function to provide the sulfonylthioureas described in EP-A-0023,422.

As an alternative to reaction 1, the sulfonyl chloride can be made from the corresponding aniline compound 7 by diazotization, then treatment with sulfur dioxide and cuprous chloride. It should be emphasised that whereas reaction 1, is written as applicable more preferably to compounds where R=CF₃ or CF₃CH₂ (i.e., to trifluoromethoxy or trifluoroethoxy compounds, the other substituents tending to give low yields), reaction 6 is applicable to compounds where R = CF₃, CH₂CF₃, or tetrahaloethyl, CHF₂ or hexafluoropropyl; thus reaction 6 is of more general applicability than reaction 1.

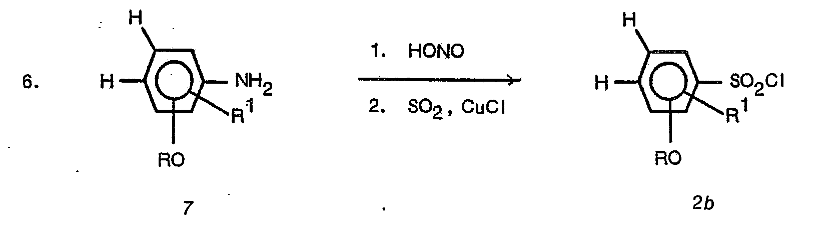

Compound 7, where R is CF₃, tetrahaloethyl, HCF₂ or hexafluoropropyl can be produced by methods described by Sheppard (loc. cit.), England (loc. cit.) and Yagupol'skii (loc. cit.). The aniline compound 7 is diazotized according to methods well known in the art, such as the addition of sodium nitrite to a hydrochloric acid solution of the compound 7. The intermediate diazonium compound is added, as in the well known Sandmeyer-type reaction, to a mixture of cuprous chloride, sulfur dioxide, and acetic acid at reduced temperature, e.g. 0 to 20°. The mixture is kept cold for to 2 hours, then is allowed to warm to ambient temperature and continue to react until nitrogen evolution has substantially stopped. Dilution with water precipitates the sulfonyl chloride, generally as an oil, which is extracted into a water-immiscible organic solvent, e.g. 1-chlorobutane, diethyl ether, toluene, ethyl acetate and the like. The organic extract can be dried and evaporated to the sulfonyl chloride or the solution can be used directly in the next step (sulfonamide preparation).

The sulfonyl chloride 2b can be used in the same sequential manner as sulfonyl chloride 2a to produce the corresponding sulfonamides, sulfonyl isocyanates and isothiocyanates, sulfonylureas, and sulfonylthioureas.

When a nitration reaction is used to make the precursor to 7, in some cases isomeric mixtures are formed, e.g.:

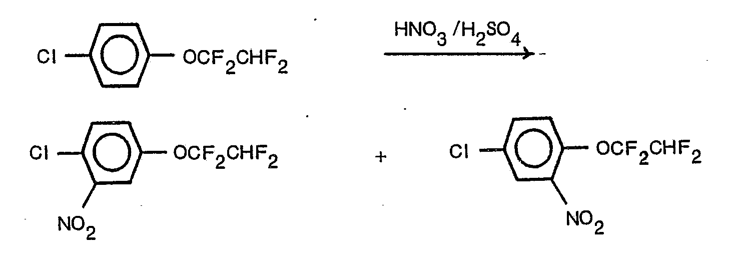

The resulting isomeric mixtures can be separated by conventional means (e.g., fractional distillation or chromatography) or used as such, in the latter case isomeric mixtures of the aniline 7, sulfonyl chloride, sulfonamide, sulfonyl isocyanate and isothiocyanate, sulfonylurea and sulfonylthiourea are found in the reactions which follow. Likewise, the isomeric mixtures of intermediates further in the synthesis sequence can be separated or used as the isomeric mixtures; and isomeric mixtures of product sulfonyl (ureas and thioureas) can be used as herbicides or separated and so used.

### (Haloalkylthio, haloalkylsulfinyl and haloalkylsulfonyl)benzenesulfonyl derivatives

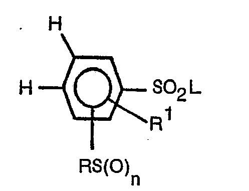

wherein the substituents are defined as for compound (I).

(Haloalkylthio, haloalkylsulfinyl and haloalkylsulfonyl)benzene derivatives 8a and 8b as starting materials for preparation of III, can be made by known methods [e.g. Chem. Abstr. 70, 96324c (1969); Chem. Abstr. 72, 66651f (1970); England, loc. cit.; Yagupol'skii, loc. cit.].

The (trifluoroethylthio)benzenes can be made by reaction of the thiophenol compounds with a trifluoroethylating agent, such as trifluoroethyl iodide or trifluoroethyl trichloromethanesulfonate. The thiophenol compound is reacted with powdered potassium hydroxide and trifluoroethyl iodide in an aprotic solvent such as DMF, dioxane or THF. The reaction process to completion at ambient temperature. Heat (e.g., with a steam bath) may be applied to increase the reaction rate. The product is isolated as described in reaction (a).

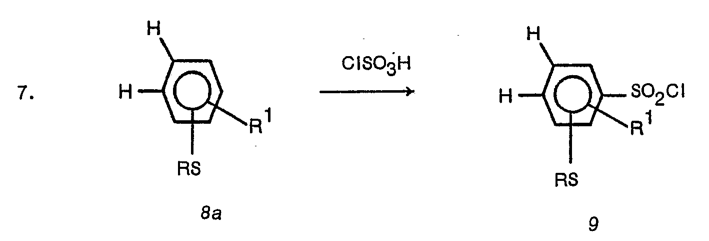

Chlorosulfonation of 8a proceeds in the same manner as described for reaction 1 for the oxygen analog 1.

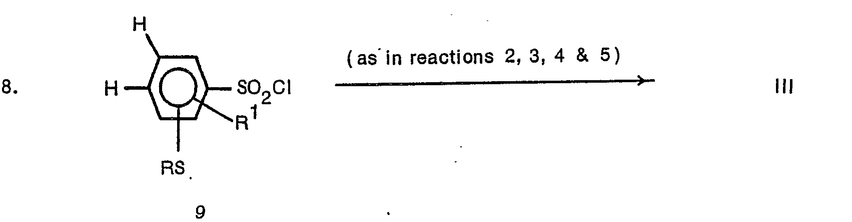

Conversion of the sulfonyl chloride sequentially to the amide, the isocyanate or isothiocyanate, and the sulfonylurea or sulfonylthiourea, proceeds as described for the oxygen analogs in reactions 2, 3, 4 and 5.

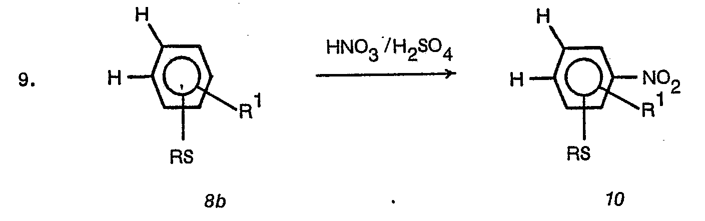

The nitration is conveniently carried out by slow addition of slightly more than 1 equivalent of 90% nitric acid to a stirred, cooled (10-30°) mixture of the sulfide 8b in sulfuric acid, stirring for an additional 10-45 minutes, pouring the reaction mixture into ice water, extracting the nitro compound into a water-immiscible organic solvent (e.g., 1-chlorobutane or methylene chloride), and evaporating the solution to leave residual nitro compound, which may be further purified by vacuum distillation. Thus, the reaction is a simple mononitration of a substituted benzene ring, a reaction well known in the art.

As mentioned for the oxygen analog, nitration of 8b can lead to isomeric nitro compounds which, likewise, can be separated or used as such.

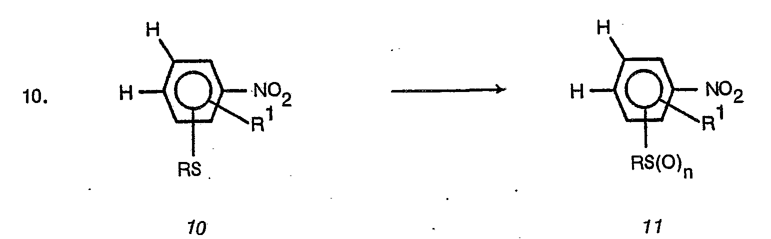

The sulfide 10 is oxidized to the sulfoxide (e.g. with 20-30% H₂0₂ in acetic acid, 1-2 hours at 90-100°); or the sulfide 10 is oxidized to the sulfone [e.g. with chromium trioxide in acetic acid at 90―110° during 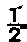 ―2 hours]. See Chem. Abstr. 70, 96324c (1969). If no oxidizing agent is used, n remains at zero and 10 = 11

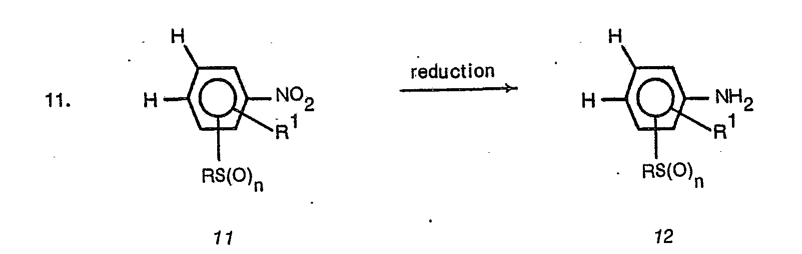

Reduction of nitrobenzene derivatives to nitroaniline derivatives is well known in the art [e.g., W. J. Hickinbottom, _{"}Reactions of Organic Compounds", 452-459, Longmans, London, 1959]. For example, the reduction can be accomplished by the portionwise addition of powdered iron to a mixture of the nitro compound in aqueous acetic acid at 60―110°; followed by dilution of the reaction mixture with water and filtering off, extracting or, when n = 110°; followed by dilution of the reaction mixture with water and filtering off, extracting or, when n = 0, steam-distilling the aniline product. Aminothiophenols can be directly tetrahaloethylated or hexafluoropropylated on the chalcogen with tetrahaloethylene or hexafluoropropene to provide directly compounds 12 with = 0 (England et al., loc. cit.; Chem. Abstr. 72,! 36584q). Also, compound 12a is commercially available (Aldrich Chemical Co., Milwaukee, Wisc.):

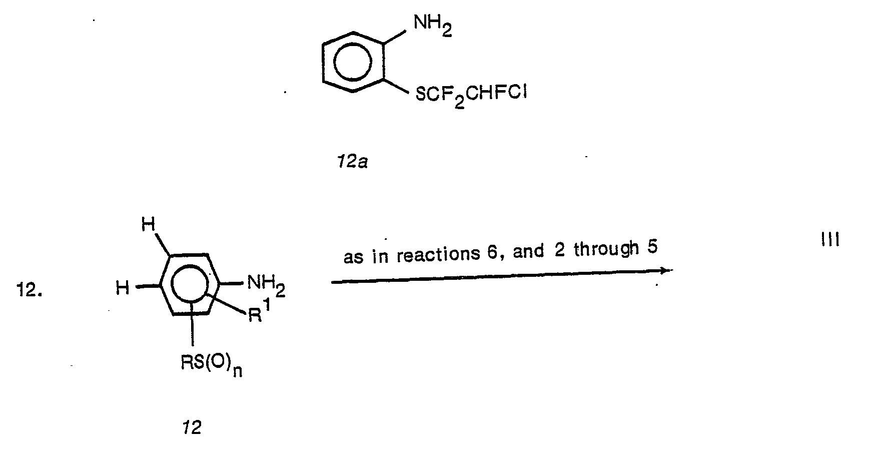

The aniline derivative 12 is diazotized and converted to the sulfonyl chloride, sulfonamide, sulfonyl isocyanate or isothiocyanate and sulfonylurea or sulfonylthiourea as described for reactions 6 and 2 through 5.

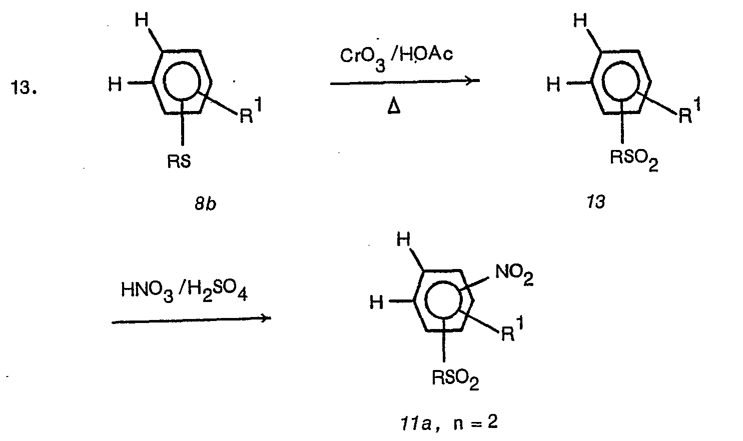

As an alternate method for preparation of the intermediate nitrosulfone (11a), the sulfide 8b is oxidized, as described for reaction 10, to the sulfone 13, followed by nitration to the nitrosulfone 11a. Nitration is accomplished with 90% nitric acid in 20% oleum at 90―100°, the nitro group joining the ring meta to the haloalkylsulfonyl group [Chem. Abstr. 53, 21766a (1959)]. The nitrosulfone 11a is converted to the sulfonylurea or sulfonylthiourea as already described (Reactions 11 6 and 2 through 5).

The following compounds of general formula (I) can be made by the processes described above. The designations "oil", "solid" or a melting or boiling point indicate that these compounds were prepared and subsequently reacted to form sulfonylureas.

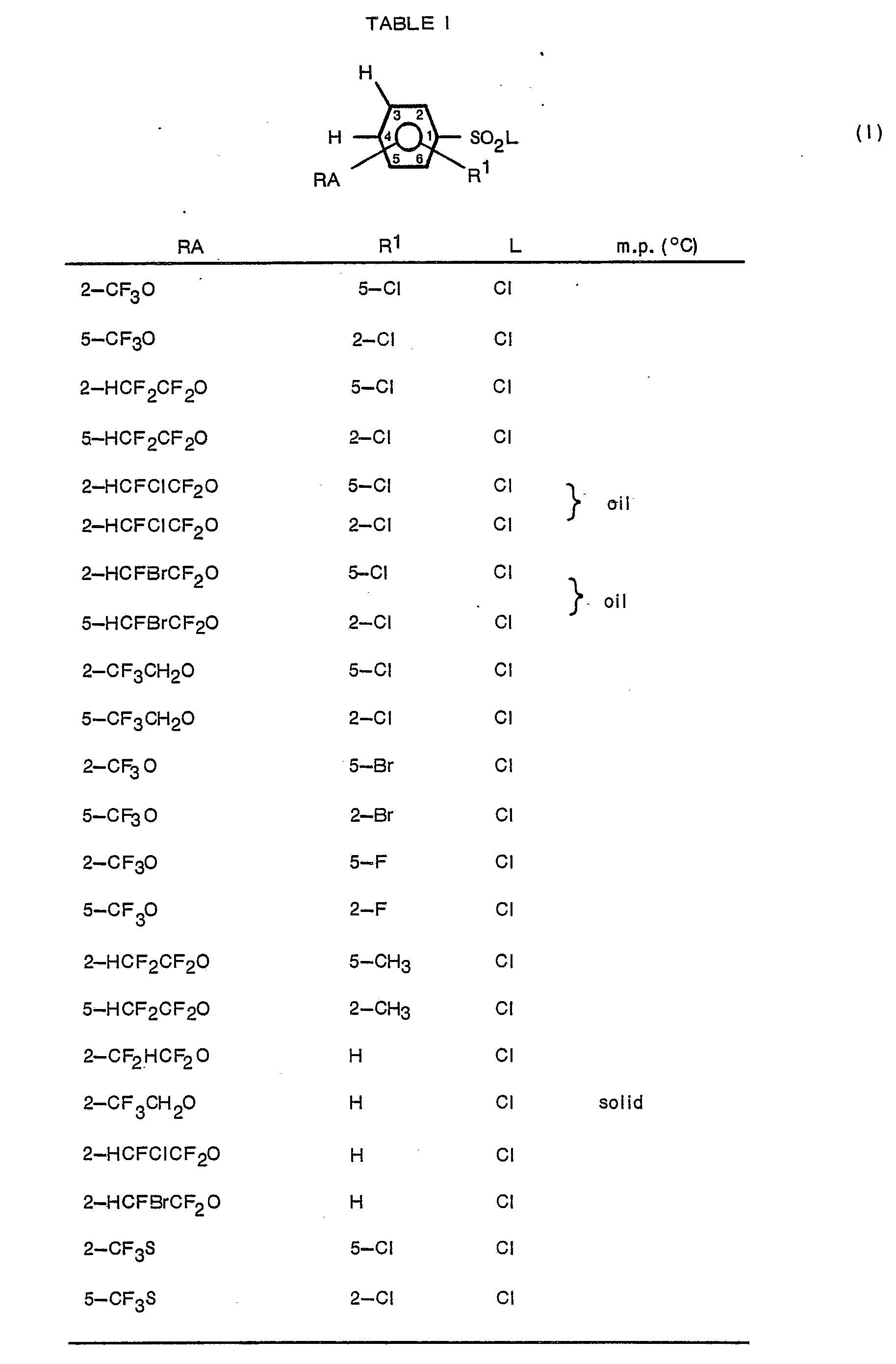

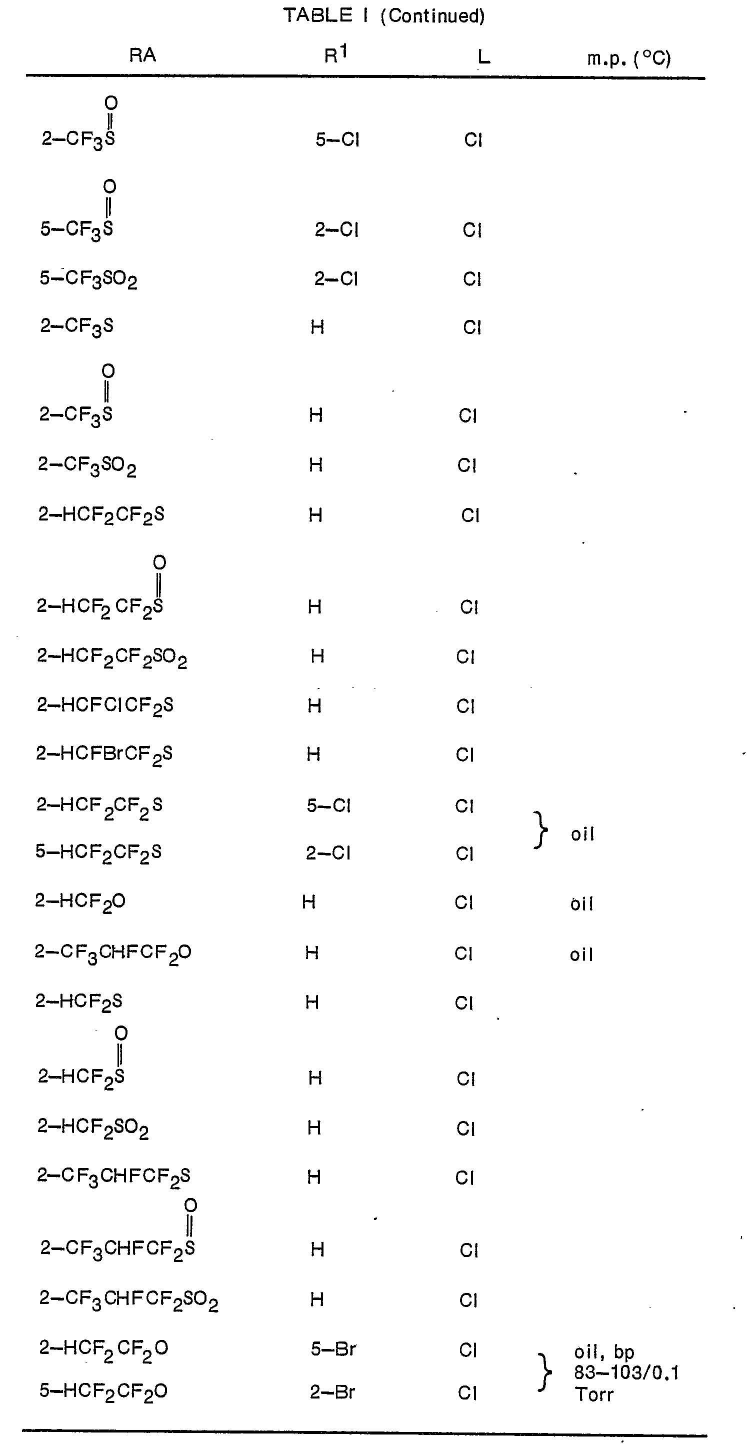

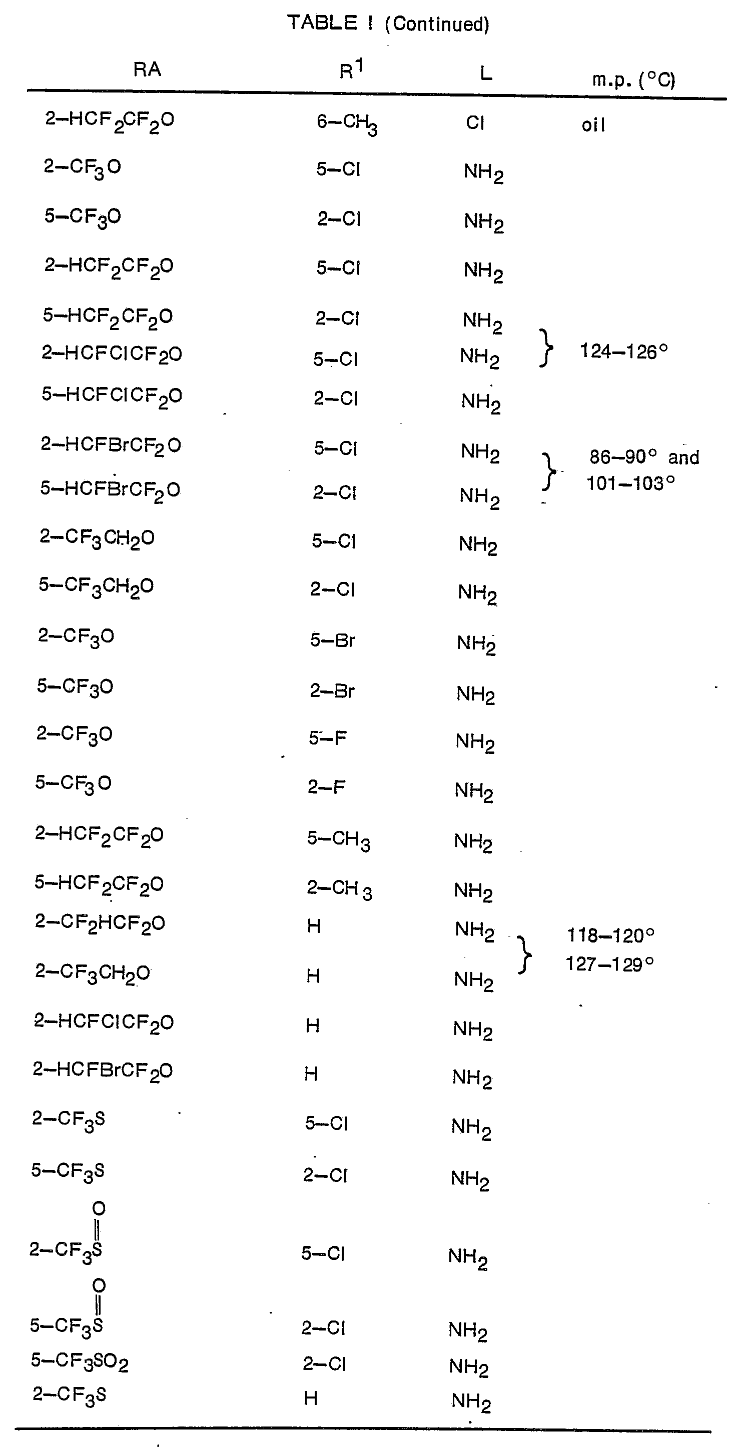

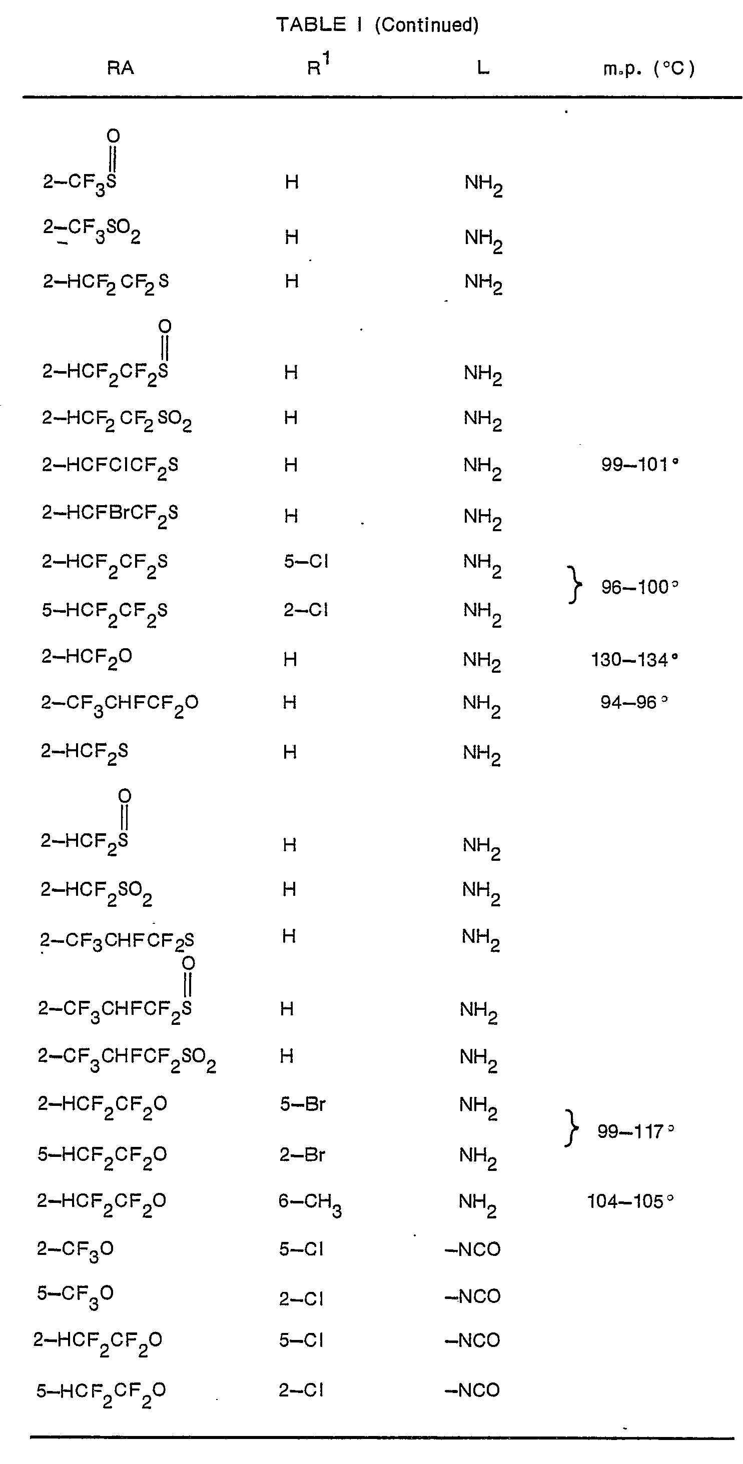

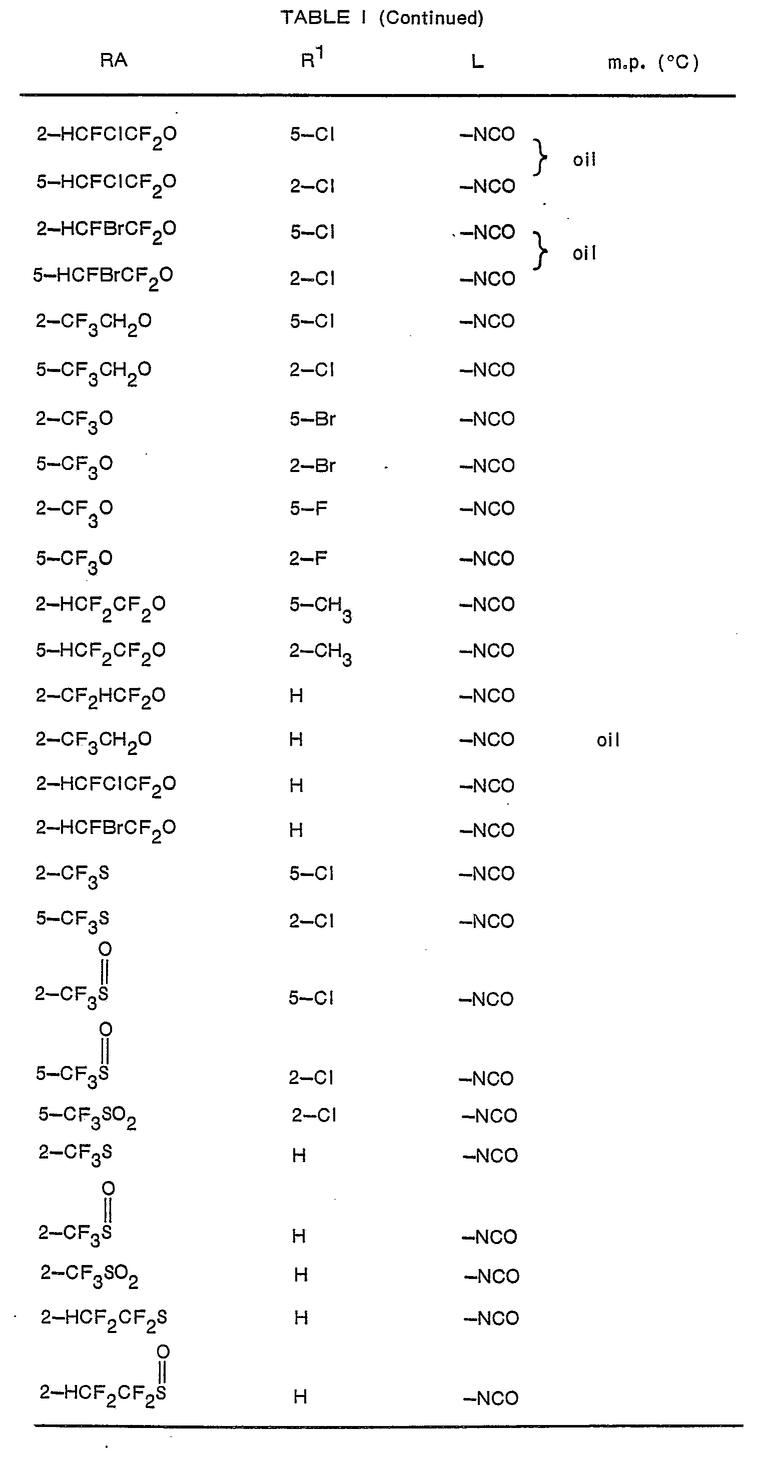

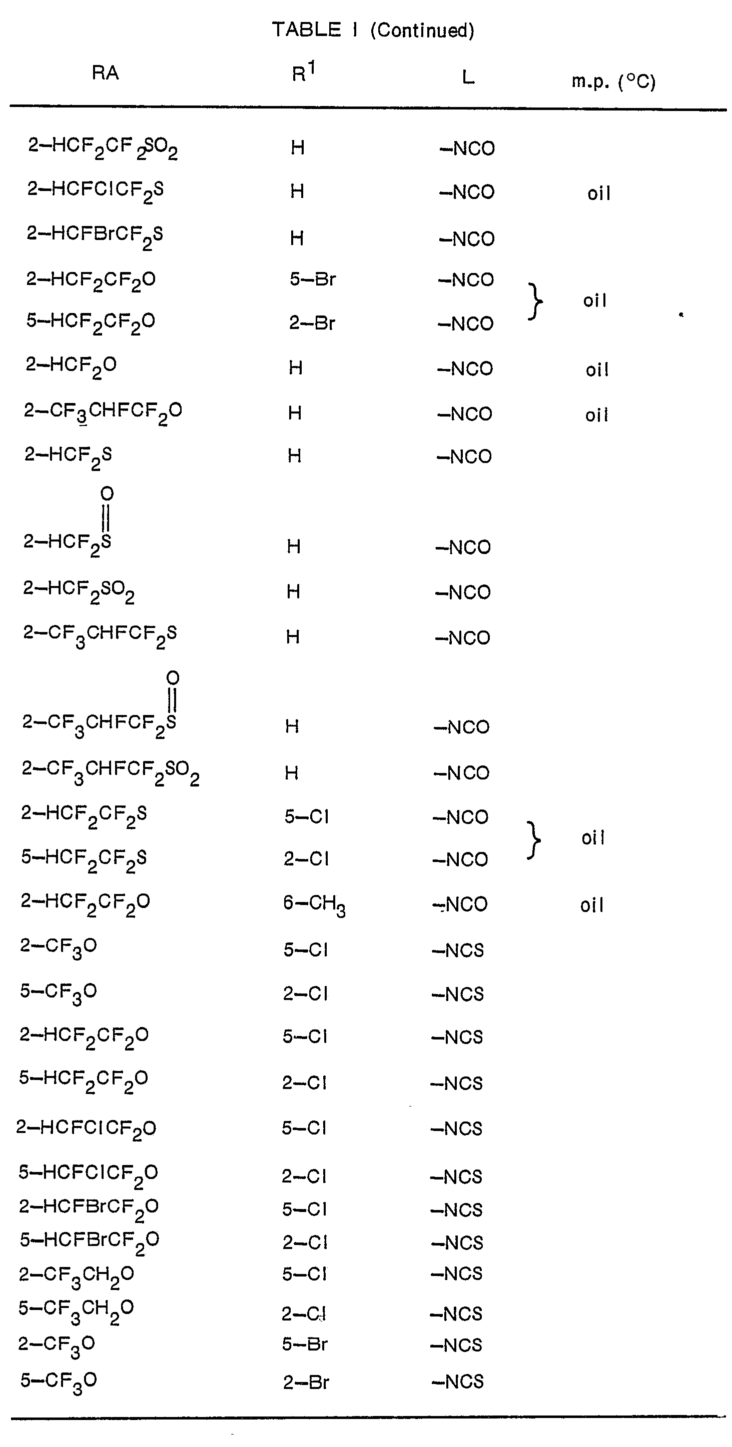

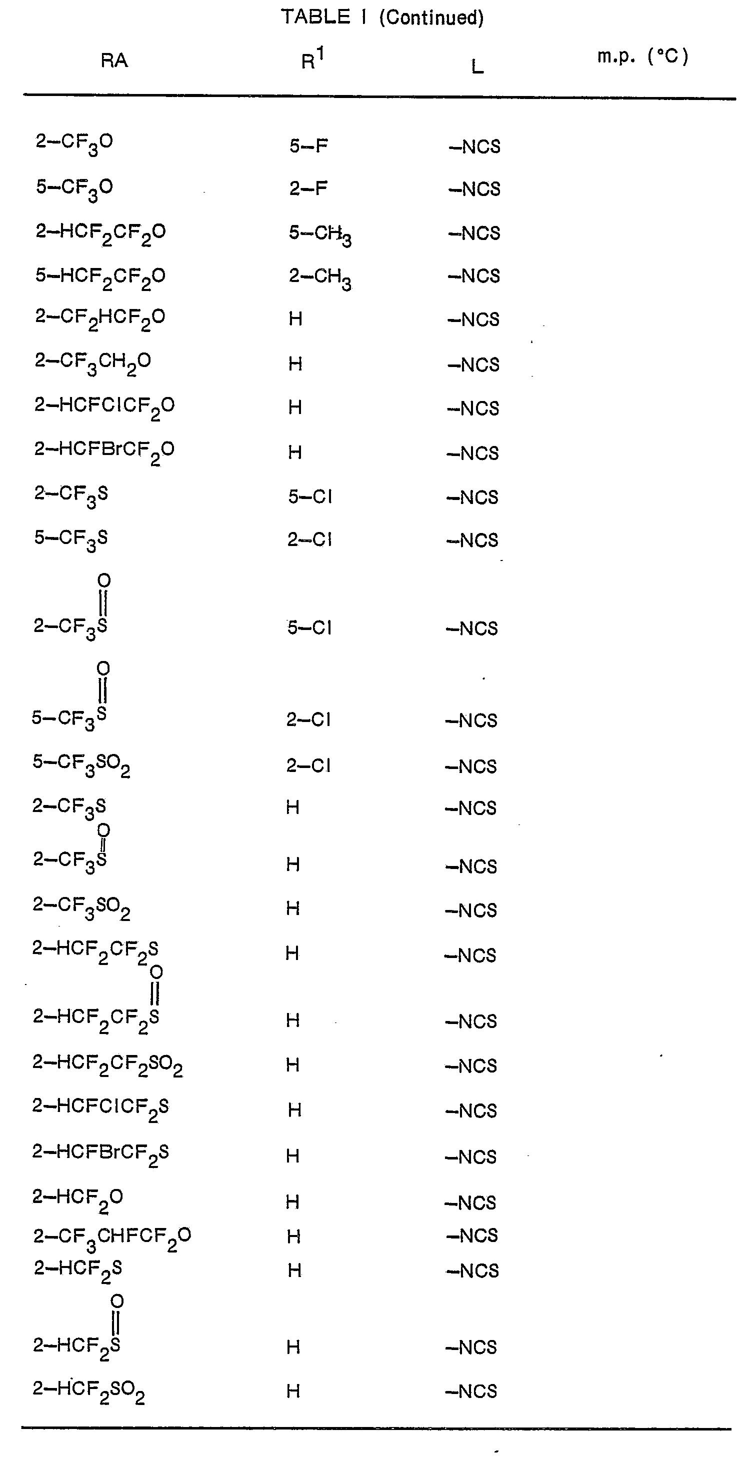

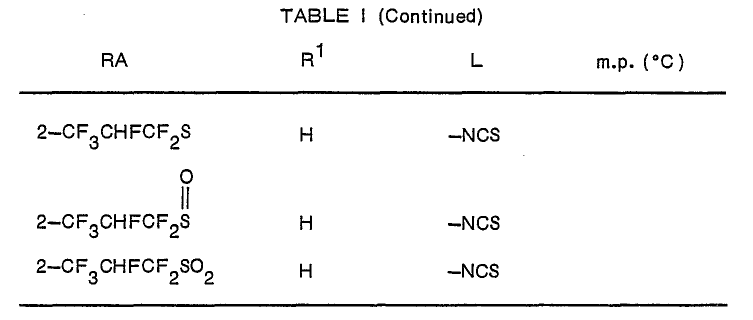

In the examples which follow, all parts and percentages are by weight and all temperatures in degrees centigrade unless specified otherwise. The examples are not to be considered as limiting, but merely exemplary of the methods which can be used to prepare the compounds of this invention.

### Example 1

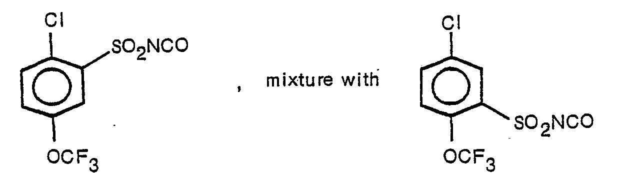

a. Into 49.1 g (0.25 mole) of 1-chloro-4-(trifluoromethoxy)benzene was dripped 82 ml (1.25 mole) of chlorosulfonic acid at 20-25°. The mixture was stirred for a day, then cooled to -10° and poured slowly onto excess, stirred ice. The mixture was extracted with butyl chloride and the butyl chloride extracts washed with water, dilute sodium bicarbonate solution, and saturated brine, dried with MgS0₄, and evaporated in vacuo to an orange oil. Vacuum distillation of the oil provided the product as an oil, bp 114-116°/8 Torr, starting material and some haloformate by-product. The oil was an isomeric mixture comprising the two isomers:

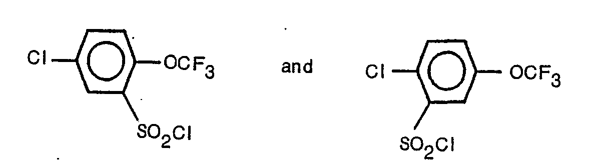

b. A solution of 26.5 g of the mixed sulfonyl chlorides in 200 ml of THF was gassed with excess ammonia at ≤30°, then evaporated and the residue treated with water and ethyl acetate. The ethyl acetate solution was washed with saturated brine, dried, concentrated to 50 ml, and diluted with hexane to provide 20 g (81%) of the product as a white solid, m.p. 93-96° (partial) and 103-105⁰ (remainder). The product was an isomeric mixture of the two isomers:

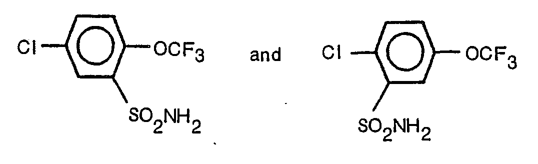

c. To 10 g of the mixed sulfonamides was added 70 ml of thionyl chloride and the mixture boiled under reflux for 20 hours. The thionyl chloride was stripped and the residual oil dissolved in 50 ml of toluene. To the solution was added 0.3 ml of pyridine and 6 ml of liquid phosgene. The resulting mixture was heated at 85-90⁰ for 2 hours under phosgene reflux. The cooled mixture was filtered and stripped to a residual oil, which was the mixed-isomer sulfonyl isocyanate (IR spectrum: strong absorption at 2240 cm-¹).

### Example 2

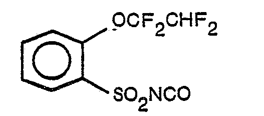

A stirred mixture of 20.9 g of 2-(1,1,2,2-tetrafluoroethoxy)benzenesulfonamide, 10 g of butyl isocyanate, 50 mg of 1,4-diazabicyclo[2.2.2]octane and 140 ml of xylene was boiled under reflux for 0.5 hour. Phosgene gas was then passed into the system under a dry-ice reflux condenser, causing the reaction temperature to fall to 120°. Phosgene addition was continued until the reaction temperature would not rise above 120°; at this point, phosgene addition was stopped. The reaction temperature was raised to 136° by removal of the dry-ice condenser and allowing phosgene to escape to a scrubber, then lowered to 25°; the mixture was then filtered. The filtrate was evaporated in vacuum to provide 2-(1,1,2,2-tetrafluoroethoxy)-benzenesulfonyl isocyanate as an oil (strong absorption at 2225 cm-¹ in the infrared spectrum, for the isocyanate), which can be further purified by vacuum distillation.

### Example 3

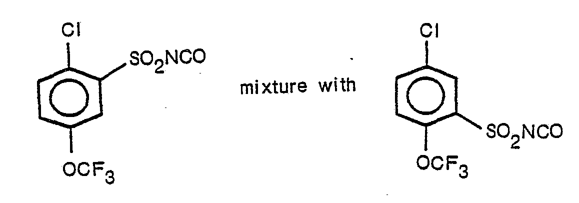

a. A solution of 68.2 g of 1-chloro-(2- and 3-nitro)-4-(trifluoromethoxy)benzene [J. Org. chem. 29, 1 (1964)] in a mixture of 229 ml of acetic acid and 57 ml of water was treated at 80-95⁰, portionwise, with 61 g of powdered iron, an additional 57 ml of water being added after half the iron was added. After an additional 30 minutes heating on a steam bath, the mixture was diluted with water and the product isolated by steam distillation, followed by extraction of the product into butyl chloride, water-washing the butyl chloride extract, evaporation and distillation at 75―79°/10 Torr. Yield: 56 g. The product, an oil, is a mixture of the two isomers:

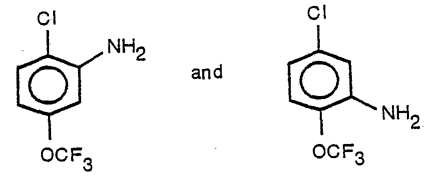

b. The aniline derivative, 56 g, is added to 200 ml of concentrated hydrochloric acid and 60 ml of acetic acid at 0° during 15 minutes. Then a solution of 26 g of sodium nitrite in 70 ml of water is added at 0 to 3° during 45 minutes. After an additional 10 minutes at 0°, the mixture is poured into a mixture of 285 ml of acetic acid, 7 g of cuprous chloride and 50 ml of sulfur dioxide at 5° over 10 minutes. After 15 minutes at 0 to 5°, the mixture is warmed to 25° and kept at 25° for 3 hours. The mixture is poured into 1.5 I of water and the sulfonyl chloride product is extracted into butyl chloride. The butyl chloride solution is washed with water, dilute sodium bicarbonate solution, and saturated brine, then dried (MgS0₄). The butyl chloride solution contains the same sulfonyl chlorides described in Example 1a, and can be converted to sulfonamides or sulfonylisocyanates as previously described.

### Example 4

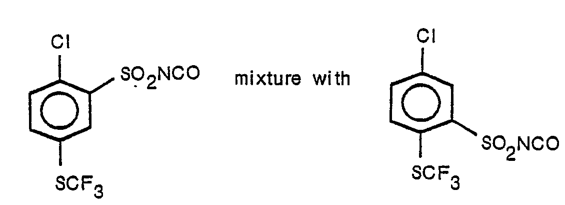

Into 53.2 g of 4-chlorophenyl trifluoromethyl sulfide is dripped 82 ml of chlorosulfonic acid at 20°. After a day, the mixture is poured onto excess ice and extracted with butyl chloride. The butyl chloride extract is washed with water and dilute sodium bicarbonate, and dried. The dried solution of isomeric sulfonyl chlorides is converted to the sulfonamides by gassing with ammonia at 25°, stripping, washing the residue with water and drying in a vacuum oven. The sulfonamides are converted to the sulfonyl isocyanate isomeric mixture as described in Example _{1c}.

### Example 5

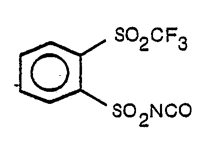

To 74 ml of concentrated hydrochloric acid and 22 ml of acetic acid is added 22.5 g of 2-aminophenyl trifluoromethyl sulfone [Chem. Abstr. 70, 96324c (1969)] at 0° over 15 minutes. A solution of 9.6 g of sodium nitrite in 26 ml of water is added at 0 to 3° over 45 minutes. After an additional 10 minutes at 0°, the reaction mixture is poured into a mixture of 107 ml of acetic acid, 2.6 g of cuprous chloride and 19 ml of sulfur dioxide at 5° over 10 minutes. The mixture is stirred at 0 to 5° for 1 hour and at 25° for 3 hours, then poured into ice water and the sulfonyl chloride extracted into ether. The ether extracts are washed with water, sodium bicarbonate solution until basic, saturated brine, and then dried with MgS0₄.

The ether solution is gassed with ammonia at 20°, then stripped. The residue is washed with water and the sulfonamide dried in a vacuum oven.

The sulfonamide is converted to the isocyanate as in Example 1c.

### Example 6

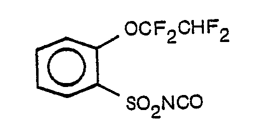

a. Into a cooled (10°) solution of 55 ml of acetic acid and 180 ml concentrated hydrochloric acid was poured 50 g of 2-(1,1,2,2-tetrafluoroethoxy)aniline. The resulting thick mixture was contacted dropwise at 0 to 5° with a solution of 23.5 g of sodium nitrite in 62 ml of water, providing an orange solution containing suspended salt. This diazonium mixture was poured, portionwise, into a stirred, cooled (0 to 5°) mixture of 45 ml S0₂ (liquid) in 257 ml of acetic acid. Gas evolution was noted. The mixture was kept at 0 to 5° for 30 minutes, then treated with 300 ml of 1-chlorobutane and 100 ml of hexane and heated to 25° during a 20- minute period. After an additional hour, water was added, the organic layer separated and washed with water (2x) and saturated sodium bicarbonate solution until a basic wash is obtained. The chlorobutane solution was dried over MgS0₄, and the filtered mixture evaporated in vacuum to 69.5 g of yellow oil, 2-(1,1,2,2-tetrafluoroethoxy)benzenesulfonyl chloride.

b. The sulfonyl chloride was dissolved in 250 ml of tetrahydrofuran (THF) and contacted at ≤30° with 14.1 ml of ammonia (liquid). After 10 minutes, the THF was evaporated in vacuum and the residual solid treated with water, and the crude sulfonamide filtered off. The sulfonamide was recrystallized from dimethylformamide/water, then 1-chlorobutane/hexane, providing 41.8 g of 2-(1,1,2,2-tetrafluoroethoxy)-benzenesulfonamide as a white solid, m.p. 117-119°. An additional portion of product was recovered from the mother liquor.

c. The 41.8 g of sulfonamide was mixed with 200 ml of thionyl chloride and the mixture boiled under reflux for 22.5 hours. The yellow solution was evaporated in vacuum and the semisolid residue treated with 150 ml of a solution of phosgene (about 15%) in toluene. Five drops of pyridine was added and the mixture heated under a dry-ice reflux condenser; phosgene was allowed to escape to a trap until the reaction temperature reached 85°. After 2 hours at 85°, the mixture was evaporated in vacuum to a residual orange oil and small amounts of solid. The residue was contacted with 1-chlorobutane, the mixture filtered and the filtrate evaporated in vacuum to an orange oil, 2-(1,1,2,2-tetrafluoroethoxy)benzenesulfonyl isocyanate.

## Claims

1. Compounds of the general formula 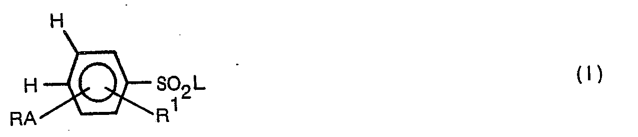 wherein
R is CF₃, CHF₂, CH₂CF₃ or CF₂CHFG, where G is F, Cl, Br or CF₃;
A is 0 or S(O)ₙ, where n is 0, 1 or 2;
R¹ is H, F, Cl, Br or CH₃; and
L is CI, NH₂, NCO or NCS.

2. Compounds of claim 1 wherein L is NCO or NCS.

3. Compounds of claim 1 wherein L is NH₂.

4. Compounds of claim 1 wherein L is CI.

5. Compounds of any of claims 1-4 wherein R is not CHF₂ and G is not CF₃.

6. Compounds of any of claims 1-5 wherein R¹ is H or CI and A is 0.

7. Compounds of any of claims 1-5 wherein R.¹ is H or Cl and A is S.

8. A compound of claim 1 which is 5-chloro-2-trifluoromethoxybenzenesulfonyl isocyanate.

9. A compound of claim 1 which is 2-chloro-5-trifluoromethoxybenzenesulfonyl isocyanate.

10. A compound of claim 1 which is 2-(1,1,2,2-tetrafluoroethoxy)benzenesulfonyl isocyanate.

11. A compound of claim 1 which is 2-difluoromethoxybenzenesulfonyl isocyanate.

12. A compound of claim 1 which is 2-trifluoromethylthio-5-chlorobenzenesulfonyl isocyanate.

13. A compound of claim 1 which is selected from
2-(1,1,3-trlfluoro-2-bromoethoxy)-5-chlorobenzenesulfonyl isocyanate;
2-chloro-5-(1,1,3-trifluoro-2-bromoethoxy)benzenesulfonyl isocyanate;
2-(2,2,2-trifluoroethoxy)benzenesulfonyl isocyanate;
2-(1,1,2-trifluoro-2-chloroethylthio)benzenesulfonyl isocyanate;
2-(1,1,2,2-tetrafluoroethoxy)-5-bromobenzenesulfonyl isocyanate;
2-bromo-5-(1,1,2,2-tetrafluoroethoxy)benzenesulfonyl isocyanate;
2-(1,1,2,2-tetrafluoroethylthio)-5-chlorobenzenesulfonyl isocyanate;
2-chloro-5-(1,1,2,2-tetrafluoroethylthio)benzenesulfonyl isocyanate; and 2-(1,1,2,2-tetrafluoroethoxy)-6-methylbenzenesulfonyl isocyanate.

14. A compound of claim 3 which is 5-chloro-2-trifluoromethoxybenzenesulfonamide.

15. A compound of claim 3 which is 2-chloro-5-trifluoromethoxybenzenesulfonamide.

16. A compound of claim 3 which is 2-(1,1,2,2-tetrafluoroethoxy)benzenesulfonamide.

17. A compound of claim 3 which is a 2-difluoromethoxybenzenesulfonamide.

18. A compound of claim 3 which is 2-(2,2,2-trifluoroethoxy)benzenesulfonamide.

19. A compound of claim 4 which is 5-chloro-2-trifluoromethoxybenzenesulfonyl chloride.

20. A compound of claim 4 which is 2-chloro-5-trifluoromethoxybenzenesulfonyl chloride.

21. A compound of claim 4 which is 2-(1,1,2,2-tetrafluoroethoxy)benzenesulfonyl chloride.

22. A compound of claim 4 which is 2-difluoromethoxybenzenesulfonyl chloride.

23. A method of making a compound of claim 1 wherein L is CI which comprises
(a) chlorosulfonating a corresponding compound of general formula 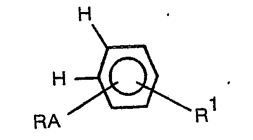 wherein R is CF₃ or CH₂CF₃, A is 0 or S and R¹ is as defined in claim 1; or
(b) diazotising an amine of general formula 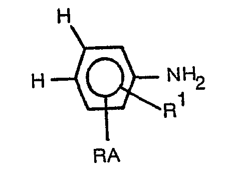 wherein R, A and R¹ are as defined in claim 1, and reacting the diazonium derivative with S0₂ and cuprous chloride.

24. A method of making a compound of claim 1 when L is NH₂ which comprises reacting a corresponding compound wherein L is CI with ammonia.

25. A method of making a compound of claim 1 wherein I is NCO or NCS which comprises:
(a) reacting a corresponding sulfonamide with SOCI₂ and subsequently with phosgene in the presence of pyridine; or
(b) reacting a corresponding sulfonamide with phosgene in the presence of an alkyl isocyanate and a base; or
(c) reacting a corresponding sulfonamide with CS₂ and potassium hydroxide and reacting the product with SOCI₂ or phosgene.

## Patentansprüche

1. Verbindungen der allgemeinen Formel 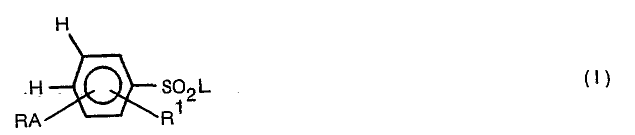 worin
R CF₃, CHF₂, CH₂CF₃ oder CF₂CHFG ist, worin G die Bedeutung von F, CI, Br oder CF₃ hat;
A 0 oder S(O)ₙ ist, worin n 0, 1 oder 2 ist;
R¹ H, F, CI, Br oder CH₃ ist; und
L CI, NH₂, NCO oder NCS ist.

2. Verbindungen nach Anspruch 1, worin L NCO oder NCS ist.

3. Verbindungen nach Anspruch 1, worin L NH₂ ist.

4. Verbindungen nach Anspruch 1, worin L CI ist.

5. Verbindungen nach einem der Ansprüche 1―4, worin R nicht CHF₂ ist und G nicht CF₃ ist.

6. Verbindungen nach einem der Ansprüche 1-5, worin R¹ H oder CI ist und A 0 ist.

7. Verbindungen nach einem der Ansprüche 1-5, worin R¹ H oder CI ist und A S ist.

8. Verbindung nach Anspruch 1, nämlich 5-Chlor-2-trifluormethoxybenzolsulfonyl-isocyanat.

9. Verbindung nach Anspruch 1, nämlich 2-Chlor-5-triflüormethoxybenzolsulfonyl-isocyanat.

10. Verbindung nach Anspruch 1, nämlich 2-(1,1,2,2-Tetrafluorethoxy)-benzolsulfonyl-isocyanat.

11. Verbindung nach Anspruch 1, nämlich 2-Difluormethoxybenzolsulfonyl-isocyanat.

12. Verbindung nach Anspruch 1, nämlich 2-Trifluormethylthio-5-chlorbenzolsulfonyl-isocyanat.

13. Verbindung nach Anspruch 1, die ausgewählt ist aus
2-(1,1,3-Trifluor-2-bromethoxy)-5-chlorbenzolsulfonyl-isocyanat;
2-Chlor-5-(1,1,3-trifluor-2-bromethoxy)-benzolsulfonyl-isocyanat;
2-(2,2,2-Trifluorethoxy)benzolsulfonyl-isocyanat;
2-(1,1,2-Trifluor-2-chlorethylthio)-benzolsulfonyl-isocyanat;
2-(1,1,2,2-Tetrafluorethoxy)-5-brombenzolsulfónyl-isocyanat;
2-Brom-5-(1,1,2,2-tetrafluorethoxy)-benzolsulfonyl-isocyanat;
2-(1,1,2,2-Tetrafluorethylthio)-5-chlorbenzolsulfonyl-isocyanat;
2-Chlor-5-(1,1,2,2-tetrafluorethylthio)-benzolsulfonyl-isocyanat; und
2-(1,1,2,2-Tetrafluorethoxy)-6-methylbenzolsulfonyl-isocyanat.

14. Verbindung nach Anspruch 3, nämlich 5-Chlor-2-trifluormethoxybenzolsulfonamid.

15. Verbindung nach Anspruch 3, nämlich 2-Chlor-5-trifluormethoxybenzolsulfonamid.

16. Verbindung nach Anspruch 3, nämlich 2-(1,1,2,2-Tetrafluorethoxy)-benzolsulfonamid.

17. Verbindung nach Anspruch 3, nämlich 2-Difluormethoxybenzolsulfonamid.

18. Verbindung nach Anspruch 3, nämlich 2-(2,2,2-Trifluorethoxy)-benzolsulfonamid.

19. Verbindung nach Anspruch 4, nämlich 5-Chlor-2-trifluormethoxybenzolsulfonyl-chlorid.

20. Verbindung nach Anspruch 4, nämlich 2-Chlor-5-trifluormethoxybenzolsulfonyl-chlorid.

21. Verbindung nach Anspruch 4, nämlich 2-(1,1,2,2-Tetrafluorethoxy)-benzolsulfonyl-chlorid.

22. Verbindung nach Anspruch 4, nämlich 2-Difluormethoxybenzolsulfonyl-chlorid.

23. Verfahren zur Herstellung einer Verbindung nach Anspruch 1, worin L CI ist, durch
(a) Chlorsulfonieren einer entsprechenden Verbindung der allgemeinen Formel 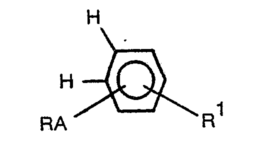 worin R CF₃ oder CH₂CF₃ ist, A O oder S ist und R¹ wie in Anspruch 1 definiert ist; oder
(b) Diazotieren eines Amins der allgemeinen Formel 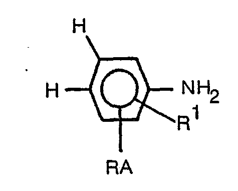 worin R, A und R¹ wie in Anspruch 1 definiert sind, und Reaktion des Diazoniumderivats mit SO₂ und Kupfer-I-chlorid.

24. Verfahren zur Herstellung einer Verbindung nach Anspruch 1, worin L NH₂ ist, durch Reaktion einer entsprechenden Verbindung, worin L CI ist, mit Ammoniak.

25. Verfahren zur Herstellung einer Verbindung nach Anspruch 1, worin L NCO oder NCS ist, durch
(a) Reaktion eines entsprechenden Sulfonamids mit SOCI₂ und anschliessend mit Phosgen, in Anwesenheit von Pyridin; oder
(b) Reaktion eines entsprechenden Sulfonamids mit Phosgen, in Anwesenheit eines Alkylisocyanats und einer Base; oder
(c) Reaktion eines entsprechenden Sulfonamids mit CS₂ und Kaliumhydroxid und Reaktion des Produkts mit SOCI₂ oder Phosgen.

## Revendications

1. Des composés de la formule générale: 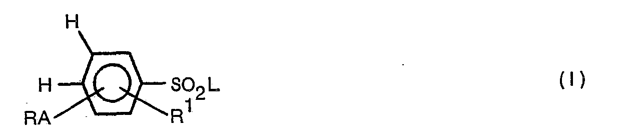 où
R est CF₃, CHF₂, CH₂CF₃ ou CF₂CHFG, où G est F, CI, Br ou CF₃.
A est 0 ou S(O)ₙ, où n est 0, 1 ou 2;
R¹ est H, F, CI, Br ou CH₃, et
L est CI, NH₂, NCO ou NCS.

2. Des composés selon la revendication 1, dans lesquels L est NCO ou NCS.

3. Des composés selon la revendication 1, dans lesquels L est NH₂.

4. Des composés selon la revendication 1, dans lesquels L est CI.

5. Des composés selon l'une quelconque des revendications 1 à 4, dans lesquels R n'est pas CHF₂ et G n'est pas CF₃.

6. Des composés selon l'une quelconque des revendications 1 à 5, dans lesquels R¹ est H ou CI et A est 0.

7. Des composés selon l'une quelconque des revendications 1 à 5, dans lesquels R¹ est H ou CI et A est S.

8. Un composé selon la revendication 1, qui est l'isocyanate de 5-chloro-2-trifluorométhoxybenzène- sulfonyle.

9. Un composé selon la revendication 1, qui est l'isocyanate de 2-chloro-5-trifluorométhoxybenzène- sulfonyle.

10. Un composé selon la revendication 1, qui est l'isocyanate de'2-(1,1,2,2-tétrafluoroéthoxy)benzène- sulfonyle.

11. Un composé selon la revendication 1, qui est l'isocyanate de 2-difluorométhoxybenzènesulfonyle.

12. Un composé selon la revendication 1, qui est l'isocyanate de 2-trifluorométhylthio-5-chloro- benzènesulfonyle.

13. Un composé selon la revendication 1, qui est choisi parmi les suivants;
isocyanate de 2-(1,1,3-trifluoro-2-bromoéthoxy)-5-chlorobenzènesulfonyle;
isocyanate de 2-chloro-5-(1,1,3-trifluoro-2-bromoéthoxy)benzènesulfonyle;
isocyanate de 2-(2,2,2-trifluoroéthoxy)benzènesulfonyle;
isocyanate de 2-(1,1,2-trifluoro-2-chloroéthylthio)benzènesulfonyle;
isocyanate de 2-(1,1,2,2-tétrafluoroéthoxy)-5-bromobenzènesuifonyle;
isocyanate de 2-bromo-5-(1,1,2,2-tétrafluoroéthoxy)benzènesulfonyle;
isocyanate de 2-(1,1,2,2-tétrafluoroéthylthio)-5-chlorobenzènesulfonyle;
isocyanate de 2-chloro-5-(1,1,2,2-tétrafluoroéthylthio)benzènesulfonyle; et
isocyanate de 2-(1,1,2,2-tétrafluoroéthoxy)-6 -méthylbenzènesulfonyle.

14. Un composé selon la revendication 3, qui est le 5-chloro-2-trifluorométhoxybenzènesulfonamide.

15. Un composé selon la revendication 3, qui est le 2-chloro-5-trifluorométhoxybenzènesulfonamide.

16. Un composé selon la revendication 3, qui est le 2-(1,1,2,2-tétrafluoroéthoxy)benzènesulfonamide.

17. Un composé selon la revendication 3, qui est un 2-difluorométhoxybenzènesulfonamide.

18. Un composé selon la revendication 3, qui est le 2-(2,2,2-trifluoroéthoxy)benzènesulfonamide.

19. Un composé selon la revendication 4, qui est le chlorure de 5-chloro-2-trifluorométhoxybenzène- sulfonyle.

20. Un composé selon la revendication 4, qui est le chlorure de 2-chloro-5-trifluorométhoxybenzène- sulfonyle.

21. Un composé selon la revendication 4, qui est le chlorure de 2-(1,1,2,2-tétrafluoroéthoxy)benzène- sulfonyle.

22. Un composé selon la revendication 4, qui est le chlorure de 2-difluorométhoxybenzènesulfonyle.

23. Un procédé de préparation d'un composé de la revendication 1 dans lequel L est CI qui comprend:
(a) la chlorosulfonation d'un composé correspondant de formule générale: 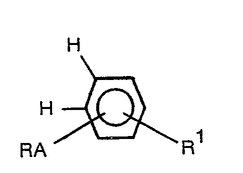 où
R est CF₃ ou CH₂CF₃,
A est 0 ou S, et
R¹ est tel que défini dans la revendication 1; ou
(b) la diazotation d'une amine de formule générale: 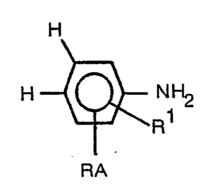 où
R, A et R¹ sont tels que définis dans la revendication 1,
et la réaction du dérivé de diazonium avec S0₂ et du chlorure cuivreux.

24. Un procédé de préparation d'un composé de la revendication 1 dans lequel L est NH₂, qui comprend la réaction d'un composé correspondant dans lequel L est CI avec de l'ammoniac.

25. Un procédé de préparation d'un composé de la revendication 1 dans lequel L est NCO ou NCS, qui comprend:
(a) la réaction d'un sulfonamide correspondant avec SOCI₂ et ensuite avec du phosgène en présence de pyridine; ou
(b) la réaction d'un sulfonamide correspondant avec du phosgène en présence d'un isocyanate d'alcoyle et d'une base; ou
(c) la réaction d'un sulfonamide correspondant avec CS₂ et de l'hydroxyde de potassium et la réaction du produit avec SOCI₂ ou du phosgène.
